# EUROPÄISCHE PATENTSCHRIFT

(11) **EP 1 342 468 B1**
(45) Veröffentlichungstag und Bekanntmachung des Hinweises auf die Patenterteilung: **25.11.2009**
(21) Anmeldenummer: 03003787.3
(22) Anmeldetag: 19.02.2003
(51) Int. Cl.: A61K 8/97, A61K 36/88

(54) **Neue kosmetische Rohstoffe aus der Pflanzenfamilie Zosteraceae für spezielle kosmetische Wirkungen und Anwendungen**
New cosmetic raw materials from plants of the family Zosteraceae for special cosmetic effects and uses
Nouvelles matières premières de plantes de la famille Zosteraceae pour effets et emplois cosmetics spécials

(30) Priorität: 20.02.2002 DE 10207172
(43) Veröffentlichungstag der Anmeldung: 10.09.2003
(73) Patentinhaber: Dötsch-Jutsch, Christel, Dr., 19069 Alt Meteln (DE)
(72) Erfinder: Dötsch-Jutsch Christel, 19069 Alt Meteln (DE); Klügel Ulrich, 12681 Berlin (DE)
(74) Vertreter: Wehlan, Helmut

(56) Entgegenhaltungen:
- DE-U- 20 008 368
- US-B1- 6 271 001
- PATENT ABSTRACTS OF JAPAN vol. 016, no. 411 (C-0979), 31. August 1992 (1992-08-31) & JP 04 139108 A (KIYOUEI KAGAKU KOUGIYOU KK), 13. Mai 1992 (1992-05-13)
- DATABASE WPI Section Ch, Week 200144 Derwent Publications Ltd., London, GB; Class B06, AN 2001-409635 XP002240713 & CN 1 291 494 A (XIN Y), 18. April 2001 (2001-04-18)

## Beschreibung

### Stand der Technik

Marine Blütenpflanzen zeichnen sich wie auch andere marine Organismen durch spezielle dem Leben im Meer angepasste Inhaltsstoffe aus (Lindequist, U. and Th. Schweder: Marine Biotechnology, in: Biotechnology. 2. ed., Ed. H.-J. Rehm Wiley-VCH Weinheim 2001, pp. 442-473). Das gilt auch für die Pflanzenfamilie der Seegrasgewächse, Zosteraceae.

Schon vor Jahrhunderten sind weltweit Seegräser aufgrund ihrer außergewöhnlichen Eigenschaften als Isolier- und Füllstoff eingesetzt worden. Zu für diese Anwendung günstigen Eigenschaften zählen vor allem die gute schall- und wärmeisolierende Wirkung, eine gute Verrottungsfestigkeit und eine Schwerentflammbarkeit. Der Einsatz von Seegras der Gattung *Zostera* in Produkten für den Einsatz als Isolier- und Dämmmaterial sowie in Produkten gegen Ungeziefer und Wanzen wurde patentrechtlich geschützt (US4016084, CA1096193). Im US-Patent Nr. 5611939 werden dem Seegras *Zostera marina* antifouling-Eigenschaften zur Verhinderung von Besatz mit marinen Bakterien und Entenmuscheln zugeschrieben, die auf der Arbeit von Todd et al (Phytochem. 34(2), 401-404, 1993) beruhen.
Die Verwendung eines Seegraspektins ist aus einem in Asien vorkommenden Seegras, *Zostera asiatica*, zur Herstellung von Erzeugnissen der Lebensmittelindustrie wie alkoholfreies Tonic (RU2125816, RU2128454) und zur Käseherstellung (RU2128918) bekannt.
Zum Stand der Technik gehört weiterhin die Verwendung grobpulverisierter oder gehäckselter Seegräser in Körperpackungen (Gebrauchsmuster DE 20008368).

Auszüge von Kräutern wurden schon seit Jahrtausenden für die Verwendung als kosmetische Zusatzstoffe und Aromen bei der Herstellung von kosmetischen Cremes, Salben und anderen Mitteln für die Schönheitspflege benutzt.
Kosmetische Rohstoffe auf der Basis von Meeresalgen finden heutzutage eine breite Anwendung und liegen weltweit im Trend der Kosmetikindustrie. Beispielhaft sind Extrakte von *Laminaria,* einer Braunalge, genannt (FR 2797187, EP 1074262), die in kosmetischen und pharmazeutischen Formulierungen für Cremes, Gele, Emulsionen oder Milch verwendet werden.
Die Verwertbarkeit von Inhaltsstoffen Mariner Höherer Blütenpflanzen wie die Pflanzenfamilie der Seegrasgewächse, Zosteraceae, für Erzeugnisse der Kosmetikindustrie ist bisher nicht weiter beschrieben worden. In der Japanischen Offenlegungsschrift JP 4139108 ("Kosmetische Stoffe") vom 13.05.1992, deren Prüfung nicht beantragt wurde, ist der Einsatz von *Zostera marina* zur Einarbeitung in Kosmetika nur angedeutet. Die Erfindung bezieht sich in der detaillierten Beschreibung ausschließlich auf die Verwendung von Meeresalgen, so daß die Verwendung von *Zostera marina* in Kosmetika in Japan sehr fraglich ist. Weiterhin umfaßt die Japanische Erfindung lediglich Wirkungen von Extrakten aus den verwendeten Algen, die im wesentlichen die Wärmehaltigkeit, Feuchtigkeit und die Reduzierung von Sommersprossen beschreiben.

### Nachteile des Standes der Technik

Obwohl der Ausgangsstoff Seegras in großem Umfang zur Verfügung steht, da er in Form von Strandgutanspülung als Rohstoffquelle kostengünstig zur Verfügung steht, ist eine breite Nutzung zur Herstellung von Produkten in der Kosmetikindustrie bisher weltweit noch in keinem Gebiet mit Seegrasvorkommen erfolgt. Eine entsprechende Anwendung war bisher aus Unkenntnis, bzw. der Verwechselung von den Zosteraceae mit diversen Makroalgen als "Seetang" und/oder "Seaweed" noch nicht möglich, so daß das wertvolle Potential dieser Pflanzenfamilie der Seegrasgewächse bisher in der Kosmetikindustrie nicht genutzt wurde. Die verwendeten Verfahren zur Trocknung von Algen wie Sprühtrocknung, Gefriertrocknung oder thermische Trocknungsmethoden sind bei Anwendung auf die Seegräser im Falle der Vorbehandlung vor der Zerkleinerung für bestimmte Anwendungen entweder zu aufwendig oder würden zum Teil dazu führen, die wertvollen Inhaltsstoffe zu zerstören, da die Zellstrukturen des Seegrases oberhalb einer Temperatur von ca. 45°C zerstört werden.

### Aufgaben der Erfindung

Aufgabe der vorliegenden Erfindung ist es, eine weitergehende Nutzung von Inhaltsstoffen der Familie Zosteraceae, insbesondere der Gattung *Zostera* zu ermöglichen, indem Extrakte, Seegras-Hyaluronate und Mikropulver aus diesen marinen Blütenpflanzen auf kostengünstigem Weg zu neuen kosmetischen Rohstoffen für die Einarbeitung in Kosmetika mit speziellen Wirkungen führen, die durch hautzellschädigende Umwelteinflüsse und alterungsbedingte Veränderungen der Haut hervorgerufen werden. Dem oben genannten Stand der Technik sind die zu entwickelnden kosmetischen Rohstoffe weit überlegen, da sie eine Verbesserung der Regenerationsfähigkeit, der biochemischen Reparatur bei kleinen Verletzungen sowie auch eine Steigerung des Hautschutzes bewirkten, was bisher nicht von den marinen Blütenpflanzen, den Seegrasgewächsen, bekannt war.

Die Aufgabe wurde gemäß den Merkmalen der Patentansprüche gelöst.

### Darlegung des Wesens der Erfindung

Aus dem natürlich vorkommenden Seegras können mit Lösungsmitteln unterschiedlicher Polarität Extrakte hergestellt werden. Als Lösungsmittel zur Herstellung von Extrakten, die die hervorragenden speziellen kosmetischen Wirkungen der biologisch aktiven Inhaltsstoffe aus Seegras bewirken, kommen n-Hexan, Ethylazetat, Methanol, Ethylether, 1,2 Propylenglycol, Propylenglycol/Wassergemisch, Aceton, Ethanol, Sonnenblumenöl, kaltes und heißes Wasser zum Einsatz.

Erfindungsgemäß sind für eine Verwendung in kosmetischen Formulierungen geeignete biologisch aktive Extrakte und für die Weiterverwendung zur Herstellung von Seegras-Hyaluronaten für spezifische kosmetische Wirkungen aus der Familie Zosteraceae durch Behandlung mit diesen Lösungsmitteln erhältlich. Erfindungsgemäß sind für die Herstellung neuer Spezialprodukte für eine Verwendung in kosmetischen Formulierungen auch mikropulverisierte Seegräser nach einer neuen von uns entwickelten Trocknungsmethode, die die wertvollen bioaktiven Inhaltsstoffe für zu zerkleinerndes Seegras belässt, möglich

Die Extrakte, Seegras-Hyaluronate und Mikropulver sind vorteilhaft erhältlich aus den Spezies *Zostera marina und*/*oder Zostera noltii.* Damit wird eine bisher nicht verwirklicht technische Lösung zur umfassenden Verwertung eines breiten Spektrums von wertvollen Inhaltsstoffen dieser marinen Blütenpflanzen erreicht. Die Extrakte, Seegras-Hyaluronate und Mikropulver können bei verschiedenen Anwendungsgebieten direkt eingesetzt werden. Es ist ebenso erfindungsgemäß, beispielsweise die Extrakte mit chromatographischen Verfahren weiter zu verarbeiten und aus diesen Salben, Cremes, Fluids, Lotionen, Gele, Hautmilch, Serum und Ampullen zu formulieren, und beispielsweise die biologisch aktiven Mikropulver in Körperwickeln, Masken und Peelings zu verwenden.

Erfindungsgemäße Extrakte werden vorteilhaft erhalten durch eine Extraktion mit einem lipophilen Lösungsmittel. Extrakte, erhältlich durch eine Extraktion mit Alkoholen, werden in besonders hohen Ausbeuten gewonnen.

Ebenso ist es erfindungsgemäß, Extrakte durch eine Wasserextraktion zu gewinnen, vorzugsweise bei Temperaturen von 10°C-80°C, wobei die Extraktion vorteilhaft stufenweise mit Wasser mit ansteigender Temperatur ausgeführt wird.

Es ist möglich, die Extraktion in mehreren Schritten mit Lösungsmitteln unterschiedlicher Polarität durchzuführen. Erfindungsgemäße Extrakte werden erhalten durch eine Extraktion des Rückstandes, der bei der Extraktion mit lipophilen Lösungsmitteln erhalten wird
Es ist vorteilhafter, möglichst hohe Anteile der Inhaltsstoffe zu extrahieren, was durch Lösungsmittelgemische erreicht wird, da die komplexe kosmetische Wirkung wie z.B. Faltenglättung und Anti-Ageing kaum den einzelnen Inhaltsstoffen zuzuordnen ist.

Die Verwendung biologisch aktiver Extrakte, Seegras-Hyaluronate und Mikropulver als vitalisierende Zusatzstoffe (Cosmeceutical) bewirken einerseits aufgrund nachgewiesener antibakterieller Wirkungen positive Effekte bei der topischen Verwendung in Kosmetika. Darüber hinaus werden die biologisch aktiven Wirkungen der Inhaltsstoffe des naturbelassenen Seegrases, die in speziellen Formulierungen während der Anwendung zur spezifischen Entfaltung kommen, in Kombination dieser günstigen Eigenschaften für pflegende, reinigende, beruhigende, lindernde, straffende, hautschützende, antioxidative sowie kräftigende und anregende Effekte genutzt.

Auf den biologisch aktiven Wirkungen der Inhaltsstoffe des Seegrases basiert die erfindungsgemäße Anwendung der neuen kosmetischen Rohstoffe in Form von Extrakten, Seegras-Hyaluronaten und Mikropulvern. In der Abbildung 1 ist die vielseitige Anwendbarkeit der Wirkstoffe/ Sekundären Pflanzenstoffe aus den neuen kosmetischen Rohstoffen zur Verwendung in Cremes, Salben, Fluids, Sprays, Gele, Lotionen, Shampoos u.m. dargestellt.

Die Erfindung soll nachstehend an einigen Beispielen erläutert werden, ohne sich auf diese Beispiele zu beschränken.

### Ausführungs- und Anwendungsbeispiele

### Beispiel 1

### Herstellung von Seegras-Hyaluronataddukten

Hyaluronate finden u.a. in der Kosmetik Verwendung, sind als Natriumsalz eine körpereigene Substanz und haben als Kosmetikrohstoff keine direkte therapeutische Wirkung. Natriumhyaluronat ist ein hochmolekularer Naturstoff, der Doppelhelices ausbildet und bei der Solvatisierung 216 Moleküle Wasser pro Monomereinheit bindet. Nach einem patentierten Verfahren (DE 19923829) können während der Solvatisierung Koordinationsstellen, v.a. durch Helixeinschlüsse von Wirkstoffen oder ölen besetzt werden. Dadurch entstehehen gelartige Addukte, die sich als Kosmetikrohstoffe ausgezeichnet bewährt haben und die gebundenen Wirkstoffe oder Öle in die Haut transportieren können, wobei das Hyaluronat als Carrier wirkt.
Zur Herstellung der Seegras-Hyaluronataddukte kann beispielhaft ein 1,2 Propylenglycol/Wasser-Gemisch als Lösungsmittel Verwendung finden.

Unter Verwendung eines 50%igen 1,2 Propylenglycol/Wasser-Gemisches werden 100 g pulverisiertes Seegras in 2 Liter dieses Lösungsmittels 1 Stunde bei 70° C eingestellt und anschließend abgesiebt und filtriert. Der so hergestellte Extrakt wird zur Herstellung eines Seegras-Hyaluronatgels weiterverarbeitet. Dieses Gel hat einen angenehmen, an Quitten erinnernden Duft und ist von opal orangebrauner Farbe. Das so erhaltene Gel kann ohne weiteren Zusatz zur topischen Anwendung kommen zur Behandlung von z.B. durch Insekten hervorgerufene Hautreize, die mit Juckreiz verbunden sind. Unter Verwendung von wässrigen Extrakten, ggfls. mit geringem Alkoholzusatz, von Seegras entstehen klare Gele. Weiße Gele entstehen beim Einarbeiten von ölextrahierten Seegrasfraktionen. Die Seegras-Hyaluronate können als Komplex weiterverarbeitet werden zur Einarbeitung in Kosmetika in Form von Cremes, Salben, Emulsionen, Lösungen oder Sprays.

Die Vorteile der Seegras-Hyaluronataddukte liegen vor allem darin, daß sie eine hohe Stabilität besitzen sowie durch die ausschließlich physikalische Bindung gegenüber chemischen Bindungen überlegen sind, so daß diese Hyaluronataddukte als neue kosmetische Rohstoffe eine Mischung bekannter zugelassener Stoffe sind, die keine gesonderten Zulassungsverfahren erfordern.

### Beispiel 2

### Herstellung von mikropulverisiertem Seegras

Die zur Herstellung beispielsweise von Körperpackungen oder Peeling-Kosmetika verwendeten üblichen Seegraspulver mit einer Korngröße von ca. 250 µm erlauben keine homogene Einarbeitung in diverse Kosmetika wie Cremes, Salben u.a.

Zur Herstellung sehr feiner Pulver (Korngröße ca. 50 µm), die für Einarbeitung in Kosmetika, bei denen eine Pigmentierung auf der Haut akzeptiert oder gewünscht wird (Packungen, Masken und Peelings, neue Produkte für die Thalassotherapie sowie ggfls. auch Kosmetika wie Cremes, Lotionen, Shampoos u.a.), geeignet sind, setzen wir ein neues Trocknungsverfahren für Seegras ein, das dazu führt, daß die Verfügbarkeit der bioaktiven Inhaltsstoffe bzw. deren Wirkungen durch die Trocknung erhalten bleibt.
Bei den sonst üblichen Verfahren werden in der Regel hohe Trocknungstemperaturen eingesetzt, die eine Schädigung der Blattstruktur des Seegrases bewirken, die dazu führt, daß die wertvollen Inhaltsstoffe zerstört werden. Der Einsatz einer neuen speziell für die Trocknung von Seegras entwickelten Apparatur, die das bei einer Schlammtrocknung bekannte "Kälteumluftverfahren" nutzt, erzielt optimale Ergebnisse, die denen einer Gefriertrocknung gleichkommen, aber bedeutend weniger aufwendig sind.
Die Mikrostruktur der Seegräser insbesondere der Blätter bleibt voll erhalten und sorgt so bei der nachfolgenden Mikrozerkleinerung und anschließenden Verwendungen für die verschiedenen Zubereitungen und Formulierungen von Kosmetik- und Wellnessprodukten für optimalen Erhalt der wertvollen Inhaltsstoffe.

### Beispiel 3

### Kosmetische Wirkungen einer Seegras-Produktserie für die Gesichtspflege

In einem Zeitraum von vier Wochen durchgeführte Tests an 15 Kundinnen mit verschiedenen Produkten einer Kosmetikserie auf Basis von Seegras-Extrakten erwiesen sich neben einer ausgezeichneten Verträglichkeit der Produkte die hervorragenden kosmetischen Wirkungen der neuen Seegras-Kosmetikprodukte.
Bei einer Maske führte das Produkt bei regelmäßiger Anwendung zu einem sichtbaren Verschwinden kleiner Falten. Die Feuchtigkeitshaltung des Produktes ist dabei optimal, so daß die erzielte Wirkung lang anhält.
Die allgemeine Hautglättung, die nach allen angewendeten Produkten zu spüren ist, macht sich besonders bei Anwendung einer Nachtcreme bemerkbar. Die Haut ist zart und geschmeidig. Die Kundinnen sahen buchstäblich am nächsten Tag den Unterschied, und die Haut wirkte ausgeruht und erfrischt und hatte sich sichtbar regeneriert.
Die sehr gute Faltenglättung in Gesichtspflegeprodukten zeigte sich besonders um die empfindliche Augenpartie. Bei Kundinnen mit Neigung zu allergischen Hautreaktionen im Augenbereich waren die Seegrasprodukte darüber hinaus ausgezeichnet verträglich.

### Beispiel 4

### Kosmetische Wirkungen einer naturbelassenen Seegras-Gesichtsmaske

Die allgemeine Hautglättung mit den Effekten der Entstehung einer zarten und geschmeidigen Haut macht sich besonders stark bei der Anwendung einer völlig naturbelassenen Maske aus mikropulverisiertem Seegras, das mit Rügener Heilkreide versetzt wurde, bemerkbar.
Bereits unmittelbar nach dem Auftragen der Maske auf die Haut entfaltet sich eine erfrischende, wohltuende und entspannende Wirkung, die je nach Schichtdicke während der Anwendungsdauer von 15 bis 25 Minuten anhält und keinerlei Nebenwirkungen hat, wie sie häufig Fertigmasken, die Kaolin enthalten, zeigen.
Da in diesem naturbelassenen Produkt keinerlei Zusätze zur Konservierung, Parfümierung und Farbgebung vorhanden sind und bei keiner der mehr als 30 getesteten Personen Irritationen an der Haut, Rötungen u. sonst. auftraten, kann davon ausgegangen werden, daß die Wirkungen von Seegras in kosmetischen Produkten u.a. stark antiseptisch und antiphlogistisch, bzw. adstringierend sind.

### Beispiel 5

### Kosmetische Effekte bei epidermalen Hautschäden

Leichte Formen von epidermalen Hautschäden, die durch Sonnenbrand oder Verbrennungen hervorgerufen werden und mit den Symptomen der Rötung der Hautoberfläche einhergehen, können mit den Seegras-Hyaluronaten oder Seegrasextrakten, die in entsprechenden Konzentrationen in Cremes, Fluids, Lotionen u.a. eingearbeitet werden, erfolgreich behandelt werden.
Da bei derartigen Hautschäden nur die äußersten Hautschichten betroffen sind, können mit den Seegrasprodukten Symptome einfacher Hautschäden kosmetisch wirksam behandelt werden und so lindernde und beruhigende Wirkungen auf die Haut ausgeübt werden.

Ein Seegras-Propylenglykolextrakt in einer eingearbeiteten Creme eignet sich ausgezeichnet zur Behandlung von rauhen Händen mit rissiger Haut, die beispielsweise durch Arbeiten mit Beton entstehen können und hier wie auch bei kleineren Hautvertetzungen wie oberflächlichen Schnittwunden, die sich in den oberen Hautschichten ereignen, zum raschen Abklingen der Beschwerden führt

Allgemein können die durch chemische, physikalische, allergische, mikrobielle Erreger wie z.B. *Staphytococcus aureus* sowie sonstige Einflüsse bei Dermatosen hervorgerufene Symptome wie Hautrötung, Schwellung, Bläschen- und Schuppenbildung u. dergl. durch begleitende Maßnahmen der Behandlung mit Seegras-Hyaluronaten und Cremes, Salben und anderen Produkten bei topischer Anwendung mit Seegrasextrakten sowie mikropulverisiertes Seegras zu einer Linderung der erkrankungsbedingten Beschwerden sowie der schnelleren Verbesserung des allgemeinen Hautbildes wie z.B. bei der Wundheilung führen. Damit sind diverse kosmetische Produkte auf Seegrasbasis geeignet, eine biochemische Reparatur von mikroskopisch kleinen Hautvertetzungen zu bewirken.

## Patentansprüche

1. Kosmetische Rohstoffe aus Seegrasgewächsen der Familie Zosteraceae, erhältlich durch
a) Extraktion von Seegras mit Lösungsmitteln und anschließendes Absieben und Filtrieren oder
b) Weiterverarbeitung des Extraktes von Anspruch 1a) zur Herstellung eines Seegras-Hyaluronatgels.

2. Kosmetische Rohstoffe nach Anspruch 1, dass es sich bei den Seegrasgewächsen um die Gattung *Zostera,* insbesondere um die Spezies *Zostera marina* und/oder *Zostera noltii* handelt.

3. Kosmetische Rohstoffe nach Anspruch 1 oder 2, **dadurch gekennzeichnet, dass** als Lösungsmittel mindestens eines der Lösungsmittel n-Hexan, Ethylacetat, Methanol, Ethylether, 1,2-Propylenglycol, Aceton, Ethanol, Sonnenblumenöl, kaltes oder heißes Wasser, Propylenglycol/Wassergemisch oder Lösungsmittelgemische eingesetzt werden.

4. Verfahren zur Herstellung von Mikropulvern aus Seegras der Familie Zosteraceae mit einer Korngröße von 50 µm, **dadurch gekennzeichnet, dass** Seegras mittels eines Kälteumluftverfahrens getrocknet und anschließend mikrozerkleinert wird.

5. Verwendung der kosmetischen Rohstoffe gemäß einem der Ansprüche 1 bis 3 oder der gemäß Anspruch 4 erhaltenen Mikropulver zur Herstellung kosmetischer Formulierungen.

6. Verwendung nach Anspruch 5 zur biochemischen Reparatur mikroskopisch kleiner Hautverletzungen und / oder epidermaler Hautschäden.

7. Verwendung nach Anspruch 5
a) zur Hautglättung und/oder
b) zur Behandlung von rauen Händen mit rissiger Haut und/oder
c) zur Vermeidung von Hautirritationen, insbesondere bei Personen mit zu Allergien neigender Haut, vor allem im Bereich der empfindlichen Augenpartie und/oder
d) zur Verringerung der Faltentiefe bei altersbedingten Hautänderungen und/oder
e) zur Verbesserung des allgemeinen Hautbildes und/oder
f) zum Schutz der Haut vor schädigenden Umwelteinflüssen **gekennzeichnet dadurch, dass** v.a. bei Kälte, Trockenheit, Sonneneinstrahlung und staubhaltiger Luft die Regenerationsfähigkeit sowie die Frische der Haut sichtbar gesteigert wird.

8. Verwendung nach Anspruch 5 zur
a) Behandlung von Insekten hervorgerufenen Hautreizen und/oder
b) Behandlung von Irritationen an der Haut und/oder
c) Behandlung von Hautrötungen, Schwellungen, Bläschen- und Schuppenbildungen
d) zur Verbesserung des Hautbildes im speziellen Fall von Cellulite.

9. Verwendung nach einem der Ansprüche 5 bis 8 in Form von Cremes, Salben, Fluids, Lotionen, Emulsionen, Lösungen, Sprays, Gele oder Shampoos.

## Claims

1. Cosmetic raw materials from sea-grass plants of the family Zosteraceae, available through
a) extraction of sea-grass with solvents and subsequently sieving and filtering or
b) processing the extract from claim 1a) for the production of a sea-grass Hyaluronat gel.

2. Cosmetic raw materials according to claim 1, wherein said that in the matter of the sea-grass plants it is the genus *Zostera,* particularly the species *Zostera marina* and/or *Zostera noltii.*

3. Cosmetic raw materials according to claim 1 or 2, thereby **characterized by** treatment with a solvent that is used at least one of the solvents n-hexane, ethyl acetate, methanol, ethyl ether, 1,2-propylene glycol, acetone, ethanol, sun-flower oil, cold or hot water, mixture of propylene glycol/water, or mixtures of solvents.

4. Technique of manufacturing micro-powders made from sea-grass of the family Zosteraceae with a particle size of 50 µm, thereby **characterized by** drying sea-grass by means of a drying-plant using cold air and subsequent micro-pulverization.

5. Utilization of the cosmetic raw materials according to one of the claims 1 to 3 or according to claim 4 the use of the obtained micro-powders for the production of cosmetic formulations.

6. Utilization according to claim 5 for the biochemical repair of microscopically small skin damages and/or epidermal skin injuries.

7. Utilization according to claim 5
a) for smoothening of the skin and/or
b) for the treatment of rough hands with a chapped skin and/or
c) for the avoidance of skin irritations particularly in persons with a tendency to skin allergies, above all around the sensitive eye area and/or
d) for the reduction in the depth of wrinkles in skin changes caused by ageing and/or
e) for the improvement of general skin appearance and/or
f) for the protection of the skin from damaging environmental influences, **characterized by** the fact that above all when exposed to cold weather, dryness, the sun and dusty air, the regeneration capability as well as the freshness of the skin are visibly increased.

8. Utilization according to claim 5
a) for the treatment of skin irritations caused by insects and/or
b) for the treatment of skin irritations and/or
c) for the treatment of skin reddening, swelling, formation of pustules and flaking
d) for the improvement of skin appearance in the special case of cellulite.

9. Utilization according to one of the claims 5 to 8 in appearance of creams, ointments, fluids, lotions, emulsions, solutions, sprays, gels or shampoos.

## Revendications

1. Matières premières cosmétiques issues de zostères marines de la famille Zosteraceae, obtenues par
a) extraction de zostère marine par produit solvant, puis par tamisage et filtrage ou
b) transformation de l'extrait de la revendication 1a) en un gel Hyaluronat de zostère.

2. Matières premières cosmétiques conformément à la revendication 1 spécifiant qu'il s'agit de zostères faisant partie du genre *Zostera,* et plus particulièrement de l'espèce *Zostera marina* et/ou *Zostera noltii.*

3. Matières premières cosmétiques conformément à la revendication 1 ou 2, **caractérisées par** l'emploi comme solvant d'au moins l'un des solvants n-hexane, acétate d'éthyle, méthanol, éther sulfurique, 1,2-propylène glycol, acétone, éthanol, huile de tournesol, eau froide ou chaude, mélange aqueux de propylène glycol ou mélange de solvants.

4. Procédé de fabrication de micropoudres de zostères de la famille Zosteraceae d'un calibre de 50 µm, **caractérisé par le fait que** la zostère a été séchée grâce à un procédé de circulation d'air froid puis micro-concassée.

5. Utilisation des matières premières cosmétiques conformément à l'une des revendications 1 à 3 ou conformément aux micropoudres obtenues d'après la revendication 4 pour la fabrication de formulations cosmétiques.

6. Utilisation conformément à la revendication 5 pour la réparation biochimique de petites blessures microscopiques de la peau et/ou de blessures de l'épiderme.

7. Utilisation conformément à la revendication 5 pour
a) le lissage de la peau et/ou
b) le traitement de mains desséchées et gercées et/ou
c) la prévention d'irritations de la peau, plus spécialement pour les personnes dont la peau a des tendances allergiques, notamment le pourtour sensible des yeux et/ou
d) la réduction de la profondeur des rides lors du vieillissement de la peau et/ou
e) l'amélioration de l'aspect général de la peau et/ou
f) la protection de la peau des effets néfastes de l'environnement, **caractérisé par** l'amélioration visible de la capacité de régénération de la peau ainsi que de sa fraîcheur en cas d'exposition au froid, à la sécheresse, au soleil et à la poussière.

8. Utilisation conformément à la revendication 5 pour
a) le traitement de réactions de la peau provoquées par les piqûres d'insectes et/ou
b) le traitement d'irritations de la peau et/ou
c) le traitement de rougeurs, enflures, formation de petites vésicules et de squames
d) l'amélioration de l'aspect général de la peau dans le cas spécifique de cellulite.

9. Utilisation conformément à l'une des revendications 5 à 8 sous la forme de crèmes, onguents, fluides, lotions, émulsions, solutions, sprays, gels ou shampoings.
